# EUROPÄISCHE PATENTSCHRIFT

(11) **EP 2 647 343 B1**
(45) Veröffentlichungstag und Bekanntmachung des Hinweises auf die Patenterteilung: **26.07.2017**
(21) Anmeldenummer: 13161214.5
(22) Anmeldetag: 27.03.2013
(51) Int. Cl.: A61B 17/12

(54) **Okklusionsvorrichtung zur Implantation innerhalb eines Aneurysmas**
Occluding device for implantation within an aneurysm
Dispositif d'occlusion destiné à être implanté à l'intérieur d'un anévrisme

(30) Priorität: 02.04.2012 DE 102012102844
(43) Veröffentlichungstag der Anmeldung: 09.10.2013
(73) Patentinhaber: Acandis GmbH & Co. KG, 76327 Pfinztal (DE)
(72) Erfinder: Cattaneo, Giorgio, 76199 Karlsruhe (DE); Baidinger, Otto, 75179 Pforzheim (DE)
(74) Vertreter: Kilchert, Jochen

(56) Entgegenhaltungen:
- WO-A1-2012/032030
- WO-A2-2006/074032
- US-A1- 2009 062 841
- US-A1- 2011 152 993

## Beschreibung

Die Erfindung betrifft eine Okklusionsvorrichtung zur Implantation innerhalb eines Aneurysmas gemäß dem Oberbegriff des Patentanspruchs 1. Ferner betrifft die Erfindung eine Anordnung mit einer derartigen Okklusionsvorrichtung. Eine Okklusionsvorrichtung ist beispielsweise aus WO 02/069783 A2 bekannt.

Bei der Behandlung von Aneurysmen wird meist das Ziel verfolgt, innerhalb des Aneurysmas die Blutströmung so zu reduzieren, dass das Blut innerhalb des Aneurysmas gerinnt. Auf diese Weise können auch bereits rupturierte Aneurysmen behandelt werden, also Aneurysmen, deren Wandung bereits an einer Stelle gebrochen ist. Durch die Bildung eines Gerinnsels innerhalb des Aneurysmas kann die Rupturstelle wieder verschlossen und die Blutung eingedämmtwerden. Üblicherweise werden zur Förderung einer Gerinnselbildung Coils eingesetzt. Dies sind dünne Drähte, die über einen Katheter in dem Aneurysma platziert werden und sich nach Entlassung aus dem Katheter willkürlich kräuseln. Dabei besteht jedoch die Gefahr, dass die Coils zumindest teilweise aus dem Aneurysma in das Blutgefäß vorragen, wodurch ebenfalls die Blutströmung innerhalb des Blutgefäßes beeinträchtigt wird. Hier kann sich ein Gerinnsel bilden, das zu einem Verschluss des Blutgefäßes führen kann. Überdies sind Coils innerhalb des Aneurysmas frei beweglich und können auch deshalb leicht aus dem Aneurysma austreten.

WO 02/069783 A2 geht einen anderen Weg in der Aneurysmenbehandlung. Demnach ist vorgesehen, ein kugelförmiges Okklusionsdevice in das Aneurysma einzuführen, wobei das Okklusionsdevice beim Zuführen über einen Katheter einen komprimierten Zustand einnimmt und nach Entlassung aus dem Katheter expandiert. Die Größe des kugelförmigen Okklusionsdevice ist kleiner als das Aneurysma selbst gewählt. Sobald das Okklusionsdevice im Aneurysma positioniert ist, wird es mit einem hochfrequenten Strom beaufschlagt, der ein Schrumpfen des Aneurysmas bewirkt. Somit legt sich die Aneurysmenwand an das kugelförmige Okklusionsdevice an. Dieser Vorgang ist relativ aufwändig und birgt die Gefahr, dass beim Schrumpfen des Aneurysmas das Okklusionsdevice aus dem Aneurysma in das Blutgefäß gedrückt wird.

Aus WO 2006/074032 A2 ist eine Okklusionsvorrichtung bekannt, die zwei Schalenelemente aufweist, welche durch ein Verbindungselement miteinander gekoppelt und zueinander relativbewegbar sind. Das Verbindungselement ist im Wesentlichen teleskopstangenartig ausgebildet und ermöglicht eine axiale Relativbewegung zwischen den beiden Schalenelementen. Durch die Relativbewegung soll erreicht werden, dass eines der Schalenelemente einen Aneurysmenhals abdeckt und das gegenüberliegende Schalenelement sich gegen den Aneurysmendom abstützt, um das abdeckende Schalenelement gegen den Aneurysmenhals abzudichten. Damit wird die Gefäßwand des Aneurysmas ungleichmäßig belastet. Besondere wird der Aneurysmendom einer höheren Kraft ausgesetzt als die Seitenwände des Aneurysmas. Das erhöht das Risiko einer Ruptur des Aneurysmas.

US 2011/012993 A1 beschreibt eine Okklusionsvorrichtung zur Behandlung von Vorhofseptumdefekten mit einem Geflecht aus Drähten, deren Drahtenden jeweils Hülsen verbunden sind, um eine insgesamt geschlossene Drahtgitterstruktur zu bilden. Ein Teil der Drahtgitterstruktur ist eingestülpt, sodass mehrere überlappende Wandungsabschnitte gebildet sind. Die einzelnen Wandungsabschnitte der Drahtgitterstruktur sind so vorgespannt, dass die Drahtgitterstruktur danach drängt, sich in den eingestülpten Zustand zu bewegen, um das Loch im Vorhofseptum zu verschließen.

Die Aufgabe der Erfindung besteht darin, eine Okklusionsvorrichtung zur Implantation innerhalb eines Aneurysmas anzugeben, die einfach handhabbar und sicher und ortsfest in einem Aneurysma positionierbar ist. Ferner besteht die Aufgabe der Erfindung darin, eine Anordnung mit einer derartigen Okklusionsvorrichtung anzugeben.

Erfindungsgemäß wird diese Aufgabe im Hinblick auf die Okklusionsvorrichtung durch den Gegenstand des Patentanspruchs 1 und im Hinblick auf die Anordnung durch den Gegenstand des Patentanspruchs 14 gelöst.

Die Erfindung beruht auf dem Gedanken, eine Okklusionsvorrichtung zur Implantation innerhalb eines Aneurysmas mit einer Wandung an zugeben, die von einem komprimierten Zustand in einen expandierten Zustand überführbar ist. Die Wandung umschließt zumindest in einem implantierten Zustand vollständig einen Hohlraum. Ferner ist die Wandung durch zwei Wandungsabschnitte gebildet, die durch ein Verbindungselement miteinander gekoppelt und zueinander relativbeweglich sind, so dass die Größe des Hohlraums variabel ist. Zumindest einer der Wandungsabschnitte weist eine schalenartige Form auf und überlappt einen anderen der Wandungsabschnitte zumindest im implantierten Zustand abschnittsweise. Die Wandungsabschnitte sind geeignet, im implantierten Zustand aufeinander zu gleiten, so dass die Wandung radial expandierbar ist. Die Okklusionsvorrichtung ist so in radialer Richtung an das Aneurysma anpassbar.

Durch die bei der Erfindung vorgesehenen zwei Wandungsabschnitte wird die Anpassbarkeit der Okklusionsvorrichtung an die Form des Aneurysmas verbessert. Konkret ermöglicht die zweigeteilte Wandung der Okklusionsvorrichtung eine Anpassung in radialer Richtung, also radial bezogen auf die Längserstreckung des Verbindungselements. Da sich die Wandungsabschnitte abschnittsweise überlappen, bleibt die Wandung insgesamt geschlossen bzw. umschließt einen Hohlraum. Die beiden Wandungsabschnitte bilden also einen Überlappungsbereich, dessen Breite variabel ist. Bei der Expansion der Wandung gleiten die beiden Wandungsabschnitte aufeinander, wodurch sich der Überlappungsbereich verkleinert. Gleichzeitig erhöht sich der Querschnittsdurchmesser der Wandung. Somit wird ermöglicht, dass das gesamte Aneurysma von der Okklusionsvorrichtung ausgefüllt wird. Dies ermöglicht eine effiziente Strömungsbeeinflussung und gleichzeitig eine Stützung der Aneurysmenwand, was weiteren Rupturen vorbeugen kann.

Erfindungsgemäß weisen die Wandungsabschnitte im Ruhezustand eine kugelsegmentartige Form auf. Die kugelsegmentartige Form ist dadurch bestimmt, dass der Wandungsabschnitt eine Öffnung aufweist, deren Öffnungsquerschnitt kleiner als der größte Querschnittsdurchmesser des Wandungsabschnitts ist. Das bedeutet, dass der Wandungsabschnitt im Wesentlichen größer ist als eine Halbkugel, bei der die Öffnung einen Durchmesser aufweist, der dem größten Querschnittsdurchmesser der Kugel entspricht. Im Rahmen der Anmeldung beinhaltet die kugelsegmentartige Form eine Halbkugelgeometrie mit über den Halbkugeldurchmesser hinausgehenden Wandungsfortsätzen. Die kugelsegmentartige Form des Wandungsabschnitts umfasst also einen maximalen Querschnittsdurchmesser, der dem Querschnittsdurchmesser einer Kugel entspricht, deren Mantelfläche teilweise durch den Wandungsabschnitt gebildet ist. Die kugelsegmentartige Form setzt sich über diesen Querschnittsdurchmesser hinweg fort und endet in einer Öffnung, die eine kreisförmige Öffnungsfläche aufweist. Die kreisförmige Öffnungsfläche umfasst einen Durchmesser, der kleiner ist als der Kugeldurchmesser.

Ferner kann vorgesehen sein, dass einer der Wandungsabschnitte zumindest im implantierten Zustand einen ersten Teilraum des Hohlraums vollständig umschließt, so dass der Hohlraum in den ersten Teilraum und einen zweiten Teilraum unterteilt ist. Mit anderen Worten kann der erste Wandungsabschnitt eine vollständig geschlossene Geometrie, beispielsweise eine Kugelform, aufweisen. Der vollständig geschlossene Wandungsabschnitt begrenzt bzw. umschließt somit einen ersten Teilraum. Der andere Wandungsabschnitt, der im Wesentlichen schalenartig geformt ist, überlappt den vollständig geschlossenen Wandungsabschnitt. Zumindest bereichsweise kann der schalenartige Wandungsabschnitt vom geschlossenen Wandungsabschnitt beabstandet sein, so dass sich zwischen den beiden Wandungsabschnitten ein zweiter Teilraum ausbildet. In beiden Fällen bleibt die radiale Flexibilität, also die Möglichkeit der Okklusionsvorrichtung, sich radial auszudehnen, unberührt.

Vorzugsweise bilden die Wandungsabschnitte im implantierten Zustand einen kreisringförmigen Überlappungsbereich. Dadurch ist eine symmetrische Ausrichtung der einzelnen Wandungsabschnitte und somit eine gleichmäßige Verteilung der Radialkraft auf die Aneurysmenwand sichergestellt. Überdies dient der kreisringförmige Überlappungsbereich als Puffer für eine weitere radiale Expansion der Wandung, wobei durch die Überlappung sichergestellt ist, dass der Hohlraum von der Wandung vollständig umschlossen wird.

Bei einer bevorzugten Ausführungsform umfassen die Wandungsabschnitte ein Geflecht aus Drähten. Ein Drahtgeflecht eignet sich besonders gut, um von einem komprimierten in einen expandierten Zustand überführt zu werden. Im komprimierten Zustand weist das Drahtgeflecht einen besonders kleinen Querschnittsdurchmesser auf, so dass die Vorrichtung auch in kleine Blutgefäße implantierbar ist.

Die Wandungsabschnitte können jeweils erste Drähte aufweisen, die in einem Zentrum des Wandungsabschnitts zentral zusammengeführt, aus der Wandungsebene heraus ausgelenkt und mit dem Verbindungselement verbunden sind. Durch das Zusammenführen der ersten Drähte und das Verbinden der zusammengeführten ersten Drähte mit dem Verbindungselement werden offene Drahtenden in dem Wandungsabschnitt vermieden. Dies reduziert die Verletzungsgefahr bei der Implantation im Aneurysma.

Die ersten Drähte können außerhalb oder innerhalb des Hohlraums zusammengeführt sein. Das Zusammenführen der ersten Drähte außerhalb des Hohlraums erleichtert die Herstellung des Wandungsabschnitts. Demgegenüber hat es bei der Anwendung der Okklusionsvorrichtung Vorteile, wenn die ersten Drähte innerhalb des Hohlraums zusammengeführt sind. Damit ist sichergestellt, dass die Drahtenden keinen Kontakt zur Aneurysmenwand finden. Eine Verletzung der Aneurysmenwand durch die Drahtenden wird somit zusätzlich vermieden.

Bei einer weiteren bevorzugten Ausführungsform ist vorgesehen, dass die Drähte, insbesondere die ersten Drähte, zumindest eines Wandungsabschnitts das Verbindungselement bilden. Mit anderen Worten können die Drähte eines Wandungsabschnitts zentral zusammengefasst und quer durch den Hohlraum geführt sein, um die beiden Wandungsabschnitte miteinander zu verbinden. Dies vereinfacht weiter den Aufbau der Okklusionsvorrichtung, da auf ein zusätzliches Bauteil als Verbindungselement verzichtet werden kann.

Ferner können die Wandungsabschnitte jeweils zweite Drähte aufweisen, die im Zentrum des Wandungsabschnitts in der Wandungsebene umgelenkt sind und jeweils eine Schlaufe bilden. Dies erhöht die Flexibilität bzw. die geometrische Anpassbarkeit der Okklusionsvorrichtung, insbesondere der Wandung. Die Schlaufen haben die Eigenschaft, dass sie sich losgelöst von dem Gittergeflecht an den Verlauf der Gefäßwand, insbesondere der Aneurysmenwand, anpassen können. Die Schlaufen erhöhen die Expansionskraft des Wandungsabschnitts, da sie eine federartige, insbesondere schenkelfederartige Wirkung entfalten.

Vorzugsweise ist das Verbindungselement vollständig innerhalb der Wandung angeordnet. Konkret ist vorgesehen, dass das Verbindungselement sich vollständig durch den Hohlraum erstreckt. Das Verbindungselement kann federartig ausgebildet sein. Auf diese Weise kann auf die beiden Wandungsabschnitte eine Vorspannung aufgebracht werden, die die Anpassung der Wandung an das Aneurysma unterstützt.

In einer weiteren bevorzugten Ausführungsform ist vorgesehen, dass das Verbindungselement ein expandierbares Element aufweist, das vollständig im Hohlraum angeordnet ist. Das expandierbare Element kann einerseits eine Vorspannfunktion umfassen, also im Wesentlichen federartig wirken. Andererseits kann das expandierbare Element innerhalb des Hohlraums zu einer Beeinflussung der Blutströmung beitragen. Auf diese Weise kann die Blutgerinnung innerhalb des Aneurysmas beschleunigt werden.

Das expandierbare Element kann ein Gittergeflecht aufweisen. Insbesondere kann das expandierbare Element aus den Drähten gebildet sein, die bereits die Wandungsabschnitte formen.

Vorzugsweise ist einer der Wandungsabschnitte zentral mit einem Abdeckelement verbunden, das im Hohlraum angeordnet ist und im Ruhezustand lose auf dem Wandungsabschnitt aufliegt. Insbesondere kann vorgesehen sein, dass das Abdeckelement dem Wandungsabschnitt zugeordnet ist, der im Gebrauch den Aneurysmenhals verschließt. Das Abdeckelement kann im Wesentlichen fluiddicht ausgebildet sein, so dass ein Nachströmen von Blut in das Aneurysma effektiv verhindert wird. Zumindest kann das Abdeckelement eine Struktur aufweisen, die eine stärkere Strömungsbeeinflussung bewirkt als der proximale Wandungsabschnitt. Das Abdeckelement ist zusätzlich flexibel, so dass das Abdeckelement unter äußerer Krafteinwirkung, beispielsweise aufgrund eines Mikrokatheters, der in das Aneurysma geschoben wird, ausweicht. Der Mikrokatheter kann somit zwischen dem Abdeckelement und dem Wandungsabschnitt in den Hohlraum geführt werden, beispielsweise um Coils im Hohlraum zu platzieren.

Gemäß einem nebengeordneten Aspekt beruht die Erfindung auf dem Gedanken, eine Anordnung mit einer Okklusionsvorrichtung und einem Transportdraht anzugeben, wobei der Transportdraht mit der Okklusionsvorrichtung lösbar verbunden ist. Der Transportdraht ist ferner innerhalb einer Katheterhülle längsverschieblich gelagert, so dass die komprimierte Okklusionsvorrichtung durch die Katheterhülle an einen Behandlungsort führbar ist. Sobald die Okklusionsvorrichtung am Behandlungsort, insbesondere in einem Aneurysma, platziert ist, kann der Transportdraht von der Okklusionsvorrichtung gelöst und über die Katheterhülle aus dem Blutgefäß entfernt werden.

Die Erfindung wird im Folgenden anhand von Ausführungsbeispielen unter Bezugnahme auf die beigefügten, schematischen Zeichnungen, näher erläutert. Darin zeigen
- Fig. 1: eine Seitenansicht der erfindungsgemäßen Okklusionsvorrichtung nach einem bevorzugten Ausführungsbeispiel im implantierten Zustand, wobei die Wandungsabschnitte aus Drähten gebildet sind, die innerhalb des Hohlraums zusammengeführt sind;
- Fig. 2: eine Seitenansicht einer erfindungsgemäßen Okklusionsvorrichtung im implantierten Zustand, wobei die die Wandungsabschnitte bildenden Drähte außerhalb des Hohlraums zusammengeführt sind;
- Fig. 3: eine Seitenansicht einer erfindungsgemäßen Okklusionsvorrichtung nach einem weiteren bevorzugten Ausführungsbeispiel, wobei die Drähte eines der Wandungsabschnitte das Verbindungselement bilden;
- Fig. 4: die Okklusionsvorrichtung nach Fig. 3, wobei die Vorrichtung in einem relativ kleineren Aneurysma eingesetzt ist;
- Fig. 5: die Okklusionsvorrichtung nach Fig. 3, die in einem relativ größeren Aneurysma eingesetzt ist;
- Fig. 6: eine Querschnittsansicht einer erfindungsgemäßen Okklusionsvorrichtung nach einem weiteren bevorzugten Ausführungsbeispiel, wobei im Hohlraum der Okklusionsvorrichtung ein zusätzliches Schirmelement vorgesehen ist;
- Fig. 7: eine Querschnittsansicht durch eine erfindungsgemäße Okklusionsvorrichtung nach einem weiteren bevorzugten Ausführungsbeispiel im implantierten Zustand, wobei einer der Wandungsabschnitte einen Teilraum vollständig umschließt;
- Fig. 8a: eine Querschnittsansicht durch eine erfindungsgemäße Okklusionsvorrichtung nach einem weiteren bevorzugten Ausführungsbeispiel, wobei ein zusätzliches Abdeckelement vorgesehen ist;
- Fig. 8b: die Okklusionsvorrichtung gemäß Fig. 8a, wobei ein Mikrokatheter in den Hohlraum geführt ist;
- Fig. 8c: die Okklusionsvorrichtung gemäß Fig. 8a, wobei über den Mikrokatheter Coils in den Hohlraum geführt werden;
- Fig. 8d: die Okklusionsvorrichtung gemäß Fig. 8a, nach Platzieren der Coils und Entfernen des Mikrokatheters;
- Fig. 9: eine Seitenansicht einer erfindungsgemäßen Okklusionsvorrichtung nach einem weiteren bevorzugten Ausführungsbeispiel im implantierten Zustand, wobei das Verbindungselement federartig ausgebildet ist;
- Fig. 10: eine Seitenansicht der erfindungsgemäßen Okklusionsvorrichtung nach einem weiteren bevorzugten Ausführungsbeispiel im implantierten Zustand, wobei der Hohlraum in zwei Teilräume unterteilt und die Wandungsabschnitte über ein federartiges Verbindungselement miteinander verbunden sind;
- Fig. 11: eine Seitenansicht einer erfindungsgemäßen Okklusionsvorrichtung nach einem weiteren bevorzugten Ausführungsbeispiel im implantierten Zustand, wobei das Verbindungselement federartige Elemente und ein expandierbares Element aufweist;
- Fig. 12: eine Seitenansicht der erfindungsgemäßen Okklusionsvorrichtung nach einem weiteren bevorzugten Ausführungsbeispiel, wobei das Verbindungselement ein expandierbares Element umfasst; und
- Fig. 13: eine Draufsicht auf die Knotenstelle eines Wandungsabschnitts, in der die ersten Drähte des Wandungsabschnitts zusammengeführt sind.

Die im Folgenden anhand von Ausführungsbeispielen beschriebenen Okklusionsvorrichtungen sind zum Implantieren in ein Aneurysma 30 vorgesehen. Damit soll erreicht werden, dass das Blut innerhalb des Aneurysmas gerinnt und somit eine Ruptur des Aneurysmas vermieden oder verschlossen wird.

Im Allgemeinen weist die Okklusionsvorrichtung eine Wandung 10 auf, die von einem komprimierten Zustand in einen expandierten Zustand überführbar ist. Im expandierten Zustand, insbesondere im Ruhezustand, d.h. ohne äußeren Krafteinfluss, umschließt die Wandung 10 einen Hohlraum 11. Der Hohlraum 11 wird auch in einem expandierten Zustand der Wandung 10 gebildet, der dem implantierten Zustand entspricht. Mit anderen Worten umschließt die Wandung 10 den Hohlraum 11 zumindest im implantierten Zustand.

Generell ist die Wandung 10 durch zwei Wandungsabschnitte 12, 13 gebildet. Die Wandungsabschnitte 12, 13 sind durch ein Verbindungselement 14 miteinander gekoppelt. Das Verbindungselement 14 bildet die Längsachse der Wandung 10. Das Verbindungselement 14 definiert somit die axiale Erstreckung der Wandung 10 bzw. der Okklusionsvorrichtung. Senkrecht zum Verbindungselement 14 ist die radiale Erstreckung der Wandung 10 bzw. der Okklusionsvorrichtung definiert.

Die beiden Wandungsabschnitte 12, 13 können als proximaler Wandungsabschnitt 12 und distaler Wandungsabschnitt 13 unterschieden werden. Der proximale Wandungsabschnitt 12 entspricht dem Abschnitt der Wandung 10, der im Gebrauch im Bereich des Aneurysmenhalses angeordnet ist. Der proximale Wandungsabschnitt 12 bildet insofern einen Verschlussabschnitt, der den Aneurysmenhals abdeckt. Der distale Wandungsabschnitt 13 ist hingegen im Gebrauch im Bereich des Aneurysmenkopfes angeordnet und stützt die Gefäßwand im Bereich des Aneurysmenkopfes. Insofern bildet der distale Wandungsabschnitt 13 einen Stützabschnitt der Okklusionsvorrichtung.

Die beiden Wandungsabschnitte 12, 13 sind zueinander relativbeweglich. Dies gilt insbesondere mit Bezug auf die radiale Erstreckung der Wandungsabschnitte 12, 13. Konkret ist die Wandung 10 durch zwei separate bzw. einzelne Wandungsabschnitte 12, 13 gebildet, die zueinander relativbeweglich sind, so dass die Größe des Hohlraums, der von der Wandung 10 umschlossen wird, variabel ist. Eine axiale Bewegung der Wandungsabschnitte 12, 13 zueinander ist durch das Verbindungselement 14 begrenzt. Die Relativbewegung zwischen den beiden Wandungsabschnitten 12, 13 erfolgt somit hauptsächlich in radialer Richtung. Dabei gleiten die sich überlappenden Bereiche der Wandungsabschnitte aufeinander. Zumindest im implantierten Zustand bleibt ein Überlappungsbereich 15 zwischen den beiden Wandungsabschnitten 12, 13 bestehen. Dadurch ist sichergestellt, dass der Hohlraum 11 vollständig geschlossen begrenzt ist.

Grundsätzlich überlappen sich die Wandungsabschnitte 12, 13 in einem implantierten Zustand der Okklusionsvorrichtung. Der implantierte Zustand entspricht dabei einem teilexpandierten Zustand. Demgegenüber wird im Rahmen der Anmeldung ein Ruhezustand unterschieden, bei dem die Okklusionsvorrichtung, insbesondere die Wandung 10, vollständig expandiert ist. Im Ruhezustand wirken auf die Wandung 10 keine äußeren Kräfte, die eine Komprimierung der Wandung 10 herbeiführen.

Im Ruhezustand können die Wandungsabschnitte 12, 13 voneinander beabstandet, also nicht-überlappend, angeordnet sein. Zwischen den Wandungsabschnitten 12, 13, kann also ein Spalt bestehen, wenn die Wandung 10 bzw. allgemein die Okklusionsvorrichtung vollständig expandiert ist, d.h. sich im Ruhezustand befindet. Die Überlappung der Wandungsabschnitte 12, 13 stellt sich vorzugsweise dann ein, wenn die Okklusionsvorrichtung nicht vollständig, sondern beispielsweise nur um 90% expandiert ist. Die Überlappung kann sich auch bei einem Expansionsgrad von 80%, insbesondere 70%, insbesondere 60%, einstellen. Mit anderen Worten können sich die Wandungsabschnitte 12, 13 überlappen, wenn die Okklusionsvorrichtung höchstens 90%, insbesondere höchstens 80%, insbesondere höchstens 70%, insbesondere höchstens 60%, des Volumens einnimmt, das dem Ruhezustand entspricht (Expansionsgrad).

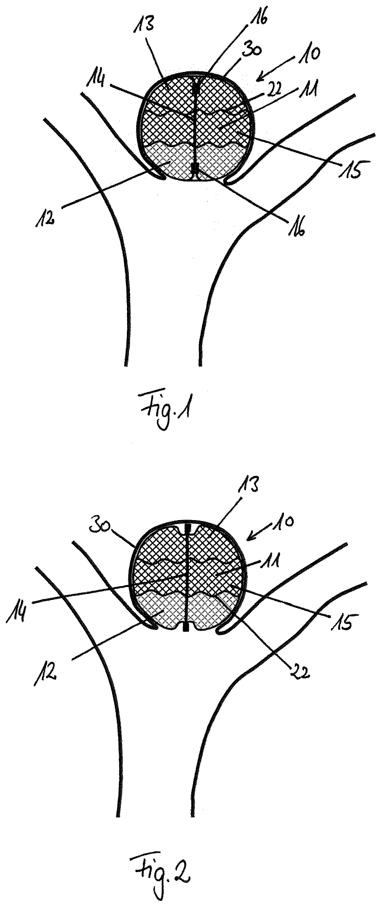

Bei dem Ausführungsbeispiel gemäß Fig. 1 ist gut erkennbar, dass die beiden Wandungsabschnitte 11, 12 im Wesentlichen schalenartig ausgebildet sind. Dabei ist darauf hinzuweisen, dass die Schalenform auch dadurch gegeben ist, dass die einzelnen Wandungsabschnitte 12, 13 im Wesentlichen eine bauchige Außenkontur aufweisen, also eine Öffnungsfläche umfassen, die kleiner als der größte Querschnittsdurchmesser des jeweiligen Wandungsabschnitts 12, 13 ist.

In Fig. 1 ist erkennbar, dass der distale Wandungsabschnitt 13 den proximalen Wandungsabschnitt 12 überlappt. Mit anderen Worten weist der proximale Wandungsabschnitt 12 einen Bereich auf, der innerhalb des distalen Wandungsabschnitts 13 angeordnet ist. Dieser Bereich entspricht dem Überlappungsbereich 15. Der Überlappungsbereich 15 ist im Wesentlichen kreisringförmig ausgebildet, wobei die Längsenden des Überlappungsbereichs 15 eine wellenartige Kontur aufweisen. Die Längsenden des Überlappungsbereichs 15 sind jeweils durch die freien Ränder 22 der beiden Wandungsabschnitte 12, 13 gebildet.

Die beiden Wandungsabschnitte 12, 13 sind jeweils durch ein Gittergeflecht gebildet. Insbesondere sind mehrere, sich regelmäßig kreuzende Drähte 19 vorgesehen, die jeweils die Wandungsabschnitte 12, 13 bilden. Zumindest ein Teil der Drähte 19 eines Wandungsabschnitts 12, 13 kann zentral zusammengeführt und durch eine Hülse 16 verbunden sein. Die Hülse 16 kann ferner dazu dienen, die Drähte 19 bzw. die Drahtenden der Drähte 19 mit dem Verbindungselement 14 zu koppeln. Das Verbindungselement 14 erstreckt sich vorzugsweise quer durch den Hohlraum 11 und ist mit dem anderen Wandungsabschnitt 13, 12 verbunden, vorzugsweise ebenfalls durch eine Hülse (Fig. 1).

Das Ausführungsbeispiel gemäß Fig. 2 entspricht im Wesentlichen dem Ausführungsbeispiel gemäß Fig. 1, wobei die Drähte 19 der einzelnen Wandungsabschnitte 12, 13 außerhalb des Hohlraums 11 zusammengeführt und jeweils über eine Hülse 16 miteinander gekoppelt sind. Bei allen Varianten kann vorgesehen sein, dass die Drähte an den freien Enden 22 der Wandungsabschnitte 12, 13, also der überlappenden Kante, umgelenkt und in das Geflecht zurückgeführt sind. Die Drähte 19 bilden also an den freien Rändern 22 jeweils atraumatische Schlaufen.

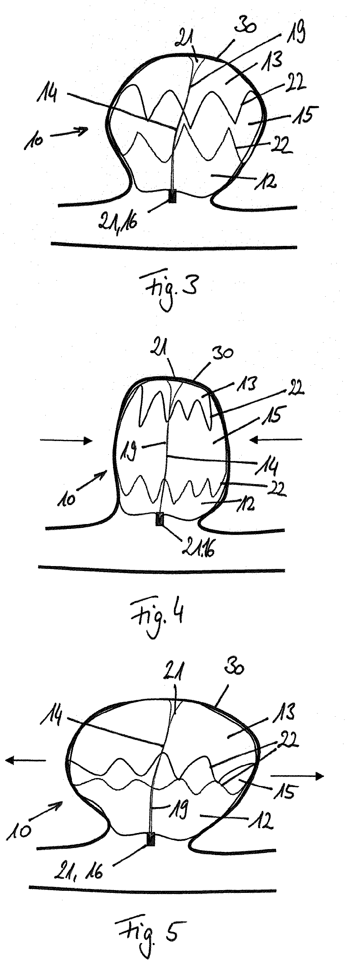

Bei den beiden Ausführungsbeispielen gemäß Fig. 1 und 2 ist erkennbar, dass sich die Okklusionsvorrichtung gut an die Form des Aneurysmas 30 anpasst. Insbesondere passt sich die Okklusionsvorrichtung in radialer Richtung an das Aneurysma an, da die Wandung 10 und folglich die einzelnen Wandungsabschnitte 12, 13 expandierbar sind. Die Wandungsabschnitte 12, 13 können sich also radial ausweiten. Dabei gleiten die Wandungsabschnitte 12, 13 aufeinander. Die folgenden Figuren 3 bis 5 zeigen anschaulich, wie sich der Überlappungsgrad, also die Größe des Überlappungsbereichs 15, bei der Anordnung in unterschiedlichen Aneurysmen 30 ändert. Gemäß Fig. 3 ist eine mittlere Größe des Überlappungsbereichs 15 dargestellt. Das Aneurysma 30 selbst weist eine annähernd kugelförmige Gestalt auf. Das Aneurysma 30 in Fig. 4 weist hingegen eine schmale langgezogene Form auf, ist also in radialer Richtung gestaucht, wie durch die beiden gegenläufigen Pfeile angedeutet ist. Somit weist die Okklusionsvorrichtung, insbesondere die Wandung 10 einen relativ größeren Überlappungsbereich 15 auf. Das bedeutet, dass die Wandung 10 radial weniger expandiert ist als in der Darstellung gemäß Fig. 3. In Fig. 5 ist die Anordnung der Okklusionsvorrichtung in einem Aneurysma 30 dargestellt, das eine im Wesentlichen flache, breite Form zeigt. Das Aneurysma 30 ist also in radialer Richtung ausgedehnt, was ebenfalls durch die beiden gegenläufig voneinander weg zeigenden Pfeile angedeutet ist. Die Okklusionsvorrichtung passt sich auch an diese Form des Aneurysmas 30 an, wobei der Überlappungsbereich 15 relativ schmal ist. Die Wandungsabschnitte 12, 13 weiten sich also in radialer Richtung aus, was dazu führt, dass der Überlappungsgrad sinkt.

Aus den Fig. 3 bis 5 ist ebenfalls erkennbar, dass die axiale Ausdehnung der Okklusionsvorrichtung durch das Verbindungselement 14 begrenzt ist. Das Verbindungselement 14 ist bei dem Ausführungsbeispiel gemäß Fig. 3 bis 5 durch die Drähte des distalen Wandungsabschnitts 13 gebildet, die in einem Zentrum 21 zusammengeführt und nach innen in den Hohlraum 11 umgelenkt sind. Die umgelenkten Drähte 19 sind durch den Hohlraum 11 geführt und in einem Zentrum 21 des proximalen Wandungsabschnitts 12 mit den Drähten des proximalen Wandungsabschnitts 12 verbunden. Die Verbindung erfolgt über eine Hülse 16.

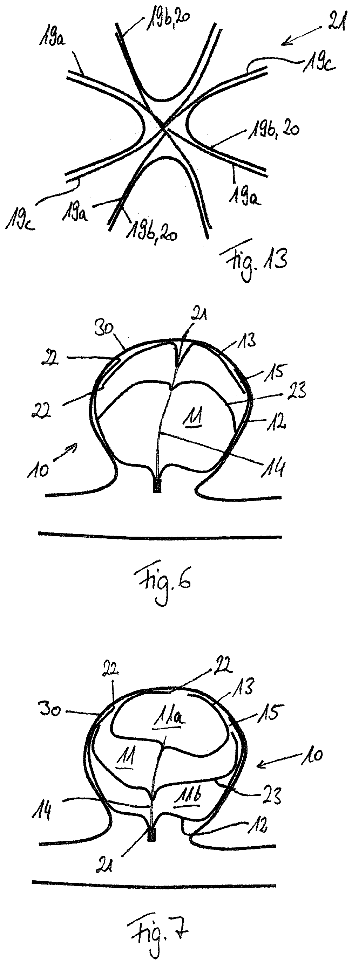

Grundsätzlich können die Wandungsabschnitte 12, 13 unterschiedliche Formen aufweisen. Beispielsweise kann der distale Wandungsabschnitt 13 eine flache schalenartige Form umfassen. Der proximale Wandungsabschnitt 12 kann hingegen eine bauchige schalenartige Form aufweisen, wie in Fig. 6 gezeigt ist. Wesentlich ist, dass die beiden Wandungsabschnitte 12, 13 derart angeordnet sind, dass sich zumindest im implantierten Zustand ein Überlappungsbereich 15 bildet.

Bei dem Ausführungsbeispiel gemäß Fig. 6 ist zusätzlich ein Schirmelement 23 vorgesehen, das im Hohlraum 11 aufgespannt ist. Das Schirmelement 23 ist zwischen dem proximalen Wandungsabschnitt 12 und dem distalen Wandungsabschnitt 13 mit dem Verbindungselement 14 verbunden. Das Verbindungselement 14 ist analog zu dem Ausführungsbeispiel gemäß Fig. 3 bis 5 durch Drähte 19 des distalen Wandungsabschnitts 13 gebildet. Das Schirmelement 23 beeinflusst die Blutströmung innerhalb des Aneurysmas 30, was zu einer beschleunigten Gerinnselbildung führt. Das Schirmelement 23 ist im Wesentlichen parallel zum distalen Wandungsabschnitt 13 gewölbt.

Fig. 7 zeigt eine weitere Variante der Okklusionsvorrichtung, wobei der distale Wandungsabschnitt 13 im Wesentlichen einen ersten Teilraum 11a des Hohlraums 11 vollständig umschließt. Der distale Wandungsabschnitt 13 bildet im implantierten Zustand im Wesentlichen einen Hohlkörper, der nahezu vollständig geschlossen ist. Der freie Rand 22 des distalen Wandungsabschnitts 13 liegt bei der Variante gemäß Fig. 7 an der Gefäßwand des Aneurysmas 30 an. Der proximale Wandungsabschnitt 12 weist eine schalenartige, insbesondere kugelsegmentartige Form auf und überlappt den distalen Wandungsabschnitt 13 teilweise. Es ist ein Überlappungsbereich 15 gebildet. Zwischen dem proximalen Wandungsabschnitt 12 und dem distalen Wandungsabschnitt 13 ist ferner ein Schirmelement 23 angeordnet, das annähernd parallel zum proximalen Wandungsabschnitt 12 gewölbt ist. Der proximale Wandungsabschnitt 12 schließt mit dem distalen Wandungsabschnitt 13 einen zweiten Teilraum 11b des Hohlraums 11 ein. Insgesamt weist die Okklusionsvorrichtung gemäß Fig. 7 im Wesentlichen eine rosenblütenartige Form auf, wobei sich einzelne Wandungsabschnitte 12 überlappen. Die freien Ränder 22 der einzelnen Wandungsabschnitte 12 können sich aufweiten und somit die radiale Anpassbarkeit der Okklusionsvorrichtung an die Aneurysmenkontur sicherstellen.

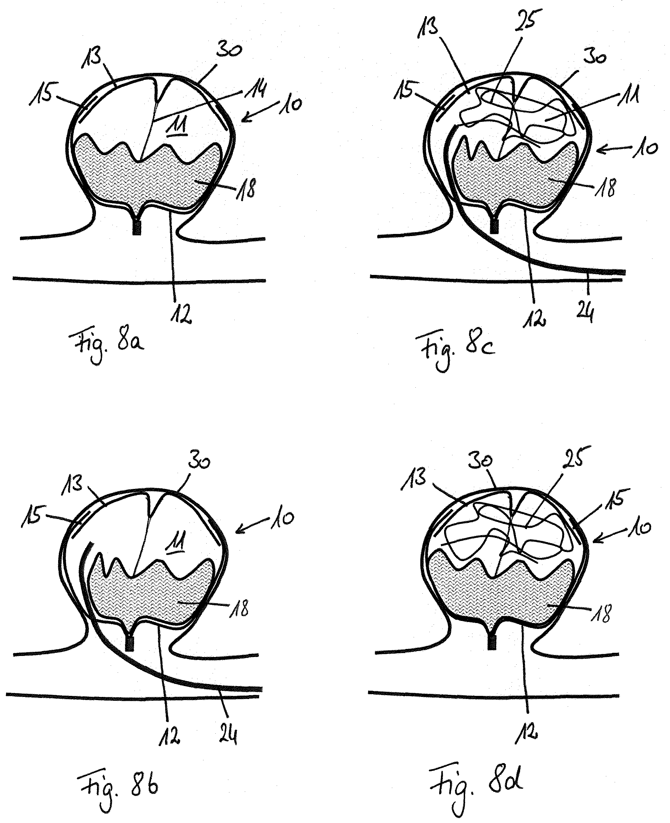

In den Fig. 8a bis 8d ist ein weiteres Ausführungsbeispiel der Okklusionsvorrichtung gezeigt. Die Wandung 10 ist aus zwei Wandungsabschnitten 12, 13 gebildet, wobei ein distaler Wandungsabschnitt 13 bzw. Stützabschnitt im Wesentlichen die Form einer flachen Schale aufweist. Der proximale Wandungsabschnitt 13 bzw. Verschlussabschnitt weist eine vergleichsweise bauchige Form auf. Im implantierten Zustand, der in den Fig. 8a bis 8d dargestellt ist, bilden die Wandungsabschnitte 12, 13 einen Überlappungsbereich 15. Im Hohlraum 11, der von den beiden Wandungsabschnitten 12, 13 umschlossen wird, ist ferner ein Abdeckelement 18 angeordnet. Das Abdeckelement 18 ist mit dem proximalen Wandungsabschnitt 12 verbunden, insbesondere im Zentrum 21. Das Abdeckelement 18 kann ebenfalls durch ein Gittergeflecht gebildet sein, wobei vorgesehen ist, dass das Abdeckelement 18 eine im Vergleich zum proximalen Wandungsabschnitt 12 feinmaschigere Gitterstruktur aufweist. Durch die feinmaschigere Gitterstruktur wird ein effizienter Verschluss des Aneurysmas 30 vom angrenzenden Blutgefäß erreicht. Das Abdeckelement 18 liegt ansonsten lose auf dem proximalen Wandungsabschnitt 12 auf. Ferner weist das Abdeckelement 18 im Wesentlichen eine flexible Struktur auf, so dass sich das Abdeckelement 18 der Form des proximalen Wandungsabschnitts 12 anpassen kann. Durch das lose aufliegende Abdeckelement 18 wird ein Zugang zum Hohlraum 11 ermöglicht. In den Fig. 8b bis 8d ist illustriert, wie beispielsweise ein Mikrokatheter 24 durch die relativ grobmaschige Struktur des proximalen Wandungsabschnitts 12 in den Hohlraum 11 geführt werden kann. Durch das Einführen des Mikrokatheters 24 wird das Abdeckelement 18 teilweise verdrängt bzw. zur Seite geschoben. Somit können über den Mikrokatheter 24 Coils 25 in den Hohlraum 11 eingebracht werden (Fig. 8c). Da das Abdeckelement 18 flexibel ist, schmiegt es sich wieder an die Innenfläche des proximalen Wandungsabschnitts 12 an, sobald der Mikrokatheter entfernt ist (Fig. 8d).

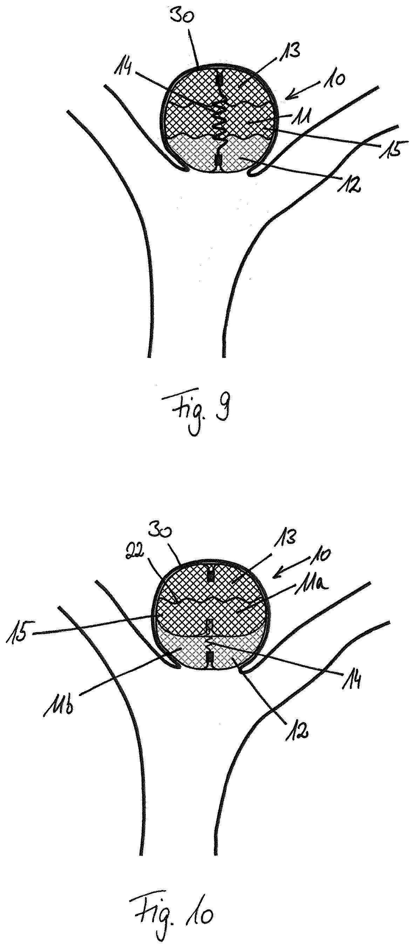

Wie zuvor bereits erläutert wurde, sind die Wandungsabschnitte 12, 13 derart flexibel, dass sich die Okklusionsvorrichtung in radialer Richtung an die Form des Aneurysmas 30 anpassen kann. Um zusätzlich eine axiale Flexibilität zu ermöglichen bzw. einen axialen Freiheitsgrad bereitzustellen, kann vorgesehen sein, dass das Verbindungselement 14 federartig ausgebildet ist. Beispielsweise kann das Verbindungselement in Form einer Spiralfeder ausgeführt sein, wie in Fig. 9 gezeigt ist. Das Ausführungsbeispiel gemäß Fig. 9 entspricht im Wesentlichen dem Ausführungsbeispiel gemäß Fig. 1 mit dem Unterschied, dass das Verbindungselement die Form einer Spiralfeder einnimmt. Dabei kann das Verbindungselement sowohl als Druckfeder, als auch als Zugfeder ausgebildet sein. Im Allgemeinen ist das Verbindungselement 14 bei dem Ausführungsbeispiel gemäß Fig. 9 federartig ausgebildet und ermöglicht eine axiale Anpassung der Okklusionsvorrichtung an die Form des Aneurysmas 30. Das Verbindungselement 14 kann, wenn es als Druckfeder wirkt, dazu beitragen, dass sich die beiden Wandungsabschnitte 12, 13 voneinander weg drücken und somit quasi im Aneurysma 30 verklemmt werden. Alternativ kann das federartige Verbindungselement 14 dafür Sorge tragen, dass sich die beiden Wandungsabschnitte 12, 13 nicht übermäßig voneinander entfernen, wobei das Verbindungselement 14 in diesem Fall als Zugfeder wirkt. Diese kann bei Aneurysmen 30 vorteilhaft sein, die einen relativ weiten Aneurysmenhals aufweisen.

In einer Abwandlung des Ausführungsbeispiels gemäß Fig. 9 kann vorgesehen sein, dass der distale Wandungsabschnitt eine vollständig geschlossene Kontur aufweist und somit einen ersten Teilraum 11a des Hohlraums 11 abtrennt. Der distale Wandungsabschnitt 13 bildet quasi einen Pfropfen, der die Öffnung des proximalen Wandungsabschnitts 12, die durch den freien Rand 22 begrenzt ist, verschließt. In jedem Fall ist zwischen dem proximalen Wandungsabschnitt 12 und dem distalen Wandungsabschnitt 13 ein Überlappungsbereich 15 gebildet, der im Wesentlichen als Wandungsreservoir dient, so dass sich die Wandung 10 an das Aneurysma 30 anpassen kann, ohne eine Lücke zwischen den beiden Wandungsabschnitten 12, 13 entstehen zu lassen. Das Verbindungselement 14 zwischen dem proximalen Wandungsabschnitt 12 und dem distalen Wandungsabschnitt 13 ist als federartiges Element ausgebildet. Dabei kann das Verbindungselement 14 ebenfalls als Druck- oder Zugfeder ausgebildet sein und entweder ein Verspannen der Okklusionsvorrichtung im Aneurysma begünstigen oder eine axiale Ausdehnung der Okklusionsvorrichtung begrenzen.

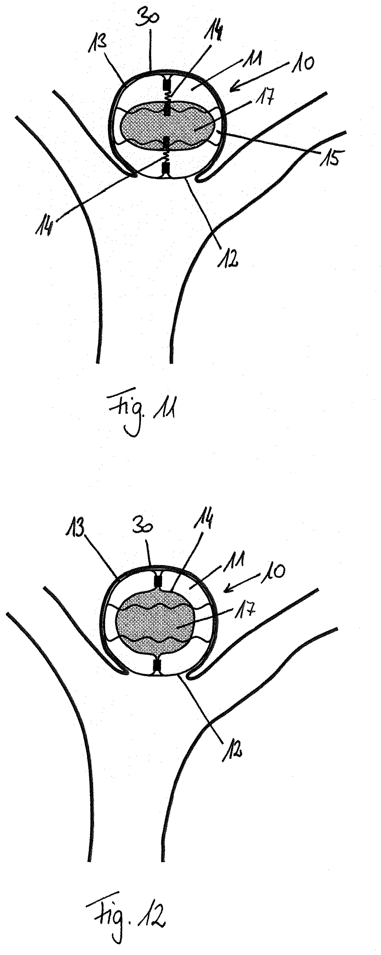

Das Verbindungselement 14 kann auch ein expandierbares Element 17 umfassen. Das expandierbare Element 17 kann beispielsweise eine im Wesentlichen kugelförmige Gitterstruktur, insbesondere ein Gittergeflecht aufweisen, wie in dem Ausführungsbeispiel gemäß Fig. 12 vorgesehen ist. Die beiden Wandungsabschnitte 12, 13 sind schalenartig ausgebildet und weisen einen Überlappungsbereich 15 auf. Die Drähte 19, die den proximalen Wandungsabschnitt 12 bilden, sind in einem Zentrum 21 zusammengeführt und mit einer Hülse 16 verbunden. Über die Hülse 16 hinaus sind die Drähte 19 weiter in das expandierbare Element 17 geführt und bilden das Gittergeflecht des expandierbaren Elements 17. Auf einer gegenüberliegenden Seite des expandierbaren Elements 17 sind die Drähte 19 wiederum zusammengeführt, mit einer Hülse fixiert und gehen im weiteren Verlauf in das Gittergeflecht des distalen Wandungsabschnitts 13 über. Das expandierbare Element 17 ermöglicht einerseits eine axiale Abstandsänderung zwischen den beiden Wandungsabschnitten 12, 13. Somit ist die Okklusionsvorrichtung auch in axialer Richtung an die Form des Aneurysmas anpassbar. Gleichzeitig füllt das expandierbare Element 17 den Hohlraum 11 teilweise aus und begünstigt somit die Blutgerinnung innerhalb des Aneurysmas 30.

Das expandierbare Element 17 ist bei dem Ausführungsbeispiel gemäß Fig. 12 direkt mit den Wandungsabschnitten 12, 13 verbunden. Alternativ kann vorgesehen sein, dass zwischen dem expandierbaren Element 17 und den Wandungsabschnitten 12, 13 jeweils ein federartiges Verbindungselement 14 vorgesehen ist. Diese Variante zeigt Fig. 11. Durch die zusätzlich vorgesehenen Verbindungselemente 14, die federartig ausgebildet sind, wird die Flexibilität der Okklusionsvorrichtung in axialer Richtung weiter gesteigert.

In Fig. 13 ist schematisch die Drahtführung im Zentrum 21 eines Wandungsabschnitts 12, 13 gezeigt. Insbesondere weist das Gittergeflecht der Wandung 10 bzw. des einzelnen Wandungsabschnitts 12, 13 erste Drähte 19a auf, die im Wesentlichen sternförmig auf das Zentrum 21 bzw. den Knotenpunkt zugeführt und aus der Wandungsebene heraus ausgelenkt sind. Die aus der Wandungsebene ausgelenkten Abschnitte der ersten Drähte 19a sind aus Gründen der Übersichtlichkeit in Fig. 13 nicht dargestellt. Fig. 13 zeigt ausschließlich die Drahtverläufe innerhalb der Wandungsebene. Die ersten Drähte 19a sind außerhalb der Wandungsebene miteinander verbunden, beispielsweise über die Hülse 16, und mit dem Verbindungselement 14 gekoppelt bzw. bilden das Verbindungselement 14. Das Drahtgeflecht bzw. Gittergeflecht weist ferner zweite Drähte 19b auf, die ebenfalls auf das Zentrum 21 bzw. den Knotenpunkt zugeführt sind. Die zweiten Drähte 19b sind im Zentrum 21 in der Wandungsebene umgelenkt und in das Geflecht zurückgeführt, so dass sich im Wesentlich Schlaufen 20 bilden. Parallel zu den zweiten Drähten 19b sind dritte Drähte 19c sternförmig auf das Zentrum 21 zugeführt und überkreuzen sich im Zentrum 21. Die dritten Drähte 19 sorgen somit für die Stabilität des jeweiligen Wandungsabschnitts 12, 13 im Bereich des Zentrums 21. Die zweiten Drähten 19b, die die Schlaufen 20 bilden, tragen zur Expansionskraft des Wandungsabschnitts 12, 13 bei. Durch die Bildung von Schlaufen 20 wird der Effekt verstärkt, dass sich der Wandungsabschnitt 12, 13 radial ausweitet und gegen die Gefäßwand des Aneurysmas 30 stemmt. Die sich im Zentrum 21 kreuzenden dritten Drähte 19c verbinden die Schlaufen 20 im Wesentlichen miteinander und ermöglichen, dass sich der Wandungsabschnitt 12, 13 radial öffnet.

### Bezugszeichenliste

- 10: Wandung
- 11: Hohlraum
- 11a: Erster Teilraum
- 11b: Zweiter Teilraum
- 12: Proximaler Wandungsabschnitt
- 13: Distaler Wandungsabschnitt
- 14: Verbindungselement
- 15: Überlappungsbereich
- 16: Hülse
- 17: Expandierbares Element
- 18: Abdeckelement
- 19: Draht
- 19a: Erster Draht
- 19b: Zweiter Draht
- 19c: Dritter Draht
- 20: Schlaufe
- 21: Zentrum
- 22: Freier Rand
- 23: Schirmelement
- 24: Mikrokatheter
- 25: Coil

## Patentansprüche

1. Okklusionsvorrichtung zur Implantation innerhalb eines Aneurysmas (30) mit einer Wandung (10), die von einem komprimierten Zustand in einen expandierten Zustand überführbar ist und geeignet ist, zumindest in einem implantierten Zustand einen Hohlraum (11) vollständig zu umschließen,
wobei die Wandung (10) durch zwei Wandungsabschnitte (12, 13) gebildet ist, die durch ein Verbindungselement (14) miteinander gekoppelt und zueinander relativbeweglich sind, wobei die Wandungsabschnitte im expandierten Zustand jeweils eine kugelsegmentartige Form aufweisen, **dadurch gekennzeichnet, dass** die Wandungsabschnitte derart beweglich sind, dass die Größe des Hohlraums (11) variabel ist, wobei die Wandungsabschnitte (12, 13) geeignet sind, sich zumindest im implantierten Zustand abschnittsweise zu überlappen und zur radialen Expansion der Wandung (10) aufeinander zu gleiten, so dass die Okklusionsvorrichtung in radialer Richtung an das Aneuryma (30) anpassbar ist.

2. Okklusionsvorrichtung nach Anspruch 1,
**dadurch gekennzeichnet, dass**
einer der Wandungsabschnitte (12, 13) geeignet ist, zumindest im implantierten Zustand einen ersten Teilraum (11a) des Hohlraums (11) vollständig zu umschließen derart, dass der Hohlraum (11) in den ersten Teilraum (11a) und einen zweiten Teilraum (11b) unterteilt ist.

3. Okklusionsvorrichtung nach einem der vorhergehenden Ansprüche,
**dadurch gekennzeichnet, dass**
die Wandungsabschnitte (12, 13) im implantierten Zustand einen kreisringförmigen Überlappungsbereich (15) bilden können.

4. Okklusionsvorrichtung nach einem der vorhergehenden Ansprüche,
**dadurch gekennzeichnet, dass**
die Wandungsabschnitte (12, 13) ein Geflecht aus Drähten (19) umfassen.

5. Okklusionsvorrichtung nach Anspruch 4,
**dadurch gekennzeichnet, dass**
die Wandungsabschnitte (12, 13) jeweils erste Drähte (19a) aufweisen, die in einem Zentrum (21) des Wandungsabschnitts (12, 13) zentral zusammengeführt, aus der Wandungsebene heraus ausgelenkt und mit dem Verbindungselement (14) verbunden sind.

6. Okklusionsvorrichtung nach Anspruch 5,
**dadurch gekennzeichnet, dass**
die ersten Drähte (19a) außerhalb oder innerhalb des Hohlraums (11) zusammengeführt sind.

7. Okklusionsvorrichtung nach einem der Ansprüche 4 bis 6,
**dadurch gekennzeichnet, dass**
die Drähte (19), insbesondere die ersten Drähte (19a), zumindest eines Wandungsabschnitts (12, 13) das Verbindungselement (14) bilden.

8. Okklusionsvorrichtung nach einem der Ansprüche 4 bis 7,
**dadurch gekennzeichnet, dass**
die Wandungsabschnitte (12, 13) jeweils zweite Drähte (19b) aufweisen, die im Zentrum (21) des Wandungsabschnitts (12, 13) in der Wandungsebene umgelenkt sind und jeweils eine Schlaufe (20) bilden.

9. Okklusionsvorrichtung nach einem der vorhergehenden Ansprüche,
**dadurch gekennzeichnet, dass**
das Verbindungselement (14) vollständing innerhalb der Wandung (10) angeordnet ist.

10. Okklusionsvorrichtung nach einem der vorhergehenden Ansprüche,
**dadurch gekennzeichnet, dass**
das Verbindungselement (14) federartig ausgebildet ist.

11. Okklusionsvorrichtung nach einem der vorhergehenden Ansprüche,
**dadurch gekennzeichnet, dass**
das Verbindungselement (14) ein expandierbares Element (17) aufweist, das vollständig im Hohlraum (11) angeordnet ist.

12. Okklusionsvorrichtung nach Anspruch 11,
**dadurch gekennzeichnet, dass**
das expandierbare Element (17) ein Gittergeflecht aufweist.

13. Okklusionsvorrichtung nach einem der vorhergehenden Ansprüche,
**dadurch gekennzeichnet, dass**
einer der Wandungsabschnitte (12, 13) zentral mit einem Abdeckelement (18) verbunden ist, das im Hohlraum (11) angeordnet ist und im Ruhezustand lose auf dem Wandungsabschnitt (12, 13) aufliegt.

14. Anordnung mit einer Okklusionsvorrichtung nach einem der vorhergehenden Ansprüche und einem Transportdraht, der mit der Okklusionsvorrichtung lösbar verbunden und innerhalb einer Katheterhülle längsverschieblich gelagert ist derart, dass die komprimierte Okklusionsvorrichtung durch die Katheterhülle an einen Behandlungsort führbar ist.

## Claims

1. An occluding device for implantation within an aneurysm (30) with a wall (10) which can be brought from a compressed state into an expanded state and at least in the implanted state is suitable for completely surrounding a hollow space (11),
wherein the wall (10) is formed by two wall sections (12, 13) which are connected to each other by a connecting element (14) and can be moved relative to each other,
wherein in the expanded state the wall sections each have a spherical segment-like shape, **characterised in that** the wall sections are movable in such a way that the size of the hollow space (11) is variable, wherein the wall sections (12, 13) are suitable, at least in the implanted state, for overlapping each other at least in sections and to slide on each other for radial expansion of the wall (10) so that the occluding device can be adapted to the aneurysm (30) in the radial direction.

2. The occluding device according to claim 1,
**characterised in that**
one of the wall sections (12, 13) is suitable, at least in the implanted state, for completely surrounding a first partial space (11a) of the hollow space (11) in such a way that the hollow space (11) is divided into the first partial space (11a) a second partial space (11b).

3. The occluding device according to one of the preceding claims,
**characterised in that**
in the implanted state the wall sections (12, 13) can form a circular overlapping area (15).

4. The occluding device according to any one of the preceding claims,
**characterised in that**
the wall sections (12, 13) comprise a mesh of wires (19).

5. The occluding device according to claim 4,
**characterised in that**
the wall sections (12, 13) each have first wires (19a) which are brought together centrally in a centre (21) of the wall section (12, 13), deflected from the wall plane and connected to the connecting element (14).

6. The occluding device according to claim 5,
**characterised in that**
the first wires (19a) are brought together outside or inside the hollow space (11).

7. The occluding device according to any one of claims 4 to 6,
**characterised in that**
the wires (19), in particular the first wires (19a) form at least one wall section (12, 13) of the connecting element (14).

8. The occluding device according to any one of claims 4 to 7,
**characterised in that**
the wall sections (12, 13) each have second wires (19b) which in the centre (21) of the wall section (12, 13) are deflected in the wall plane and each form a loop (20).

9. The occluding device according to any one of the preceding claims,
**characterised in that**
the connecting element (14) is arranged completely within the wall (10).

10. The occluding device according to any one of the preceding claims,
**characterised in that**
the connecting element (14) is designed in spring-like manner.

11. The occluding device according to any one of the preceding claims,
**characterised in that**
the connecting element (14) comprises an expandable element (17) which is arranged completely within the hollow space (11).

12. The occluding device according to claim 11,
**characterised in that**
the expanded element (17) comprises a grid mesh.

13. The occluding device according to any one of the preceding claims,
**characterised in that**
one of the wall sections (12, 13) is centrally connected with a cover element (18) which is arranged in the hollow space (11) and in the resting state lies loose on the wall section (12, 13).

14. An arrangement with an occluding device according to any one of the preceding claims and a transport wire which is detachably connected to the occluding device and is borne within a catheter sheath in a longitudinally displaceable manner in such a way that the compressed occluding device can be moved to a treatment location through the catheter sheath.

## Revendications

1. Dispositif d'occlusion destiné à être implanté à l'intérieur d'un anévrisme (30) comprenant une paroi (10) qui peut être amenée d'un état comprimé à un état expansé et est appropriée pour entourer complètement une cavité (11), au moins dans un état implanté,
dans lequel la paroi (10) est formée par deux tronçons de paroi (12, 13) qui sont couplés entre eux par un élément de liaison (14) et mobiles l'un par rapport à l'autre,
dans lequel les tronçons de paroi présentent dans l'état expansé une forme semblable à des segments sphériques, **caractérisé en ce que** les tronçons de paroi sont ainsi mobiles que la taille de la cavité (11) est variable, dans lequel les tronçons de paroi (12, 13) sont appropriés pour se chevaucher par tronçons au moins dans l'état implanté, et pour glisser l'un sur l'autre en vue de l'expansion radiale de la paroi, de façon à ce que le dispositif d'occlusion soit adaptable à l'anévrisme (30) dans le sens radial.

2. Dispositif d'occlusion selon la revendication 1,
**caractérisé en ce**
**qu'**un des tronçons de paroi (12, 13) est approprié, au moins dans l'état implanté, pour entourer complètement un premier espace partiel (11a) de la cavité (11), de telle sorte que la cavité (11) est divisée en le premier espace partiel (11a) et un second espace partiel (11b).

3. Dispositif d'occlusion selon l'une des revendications précédentes,
**caractérisé en ce que**
les tronçons de paroi (12, 13) peuvent former une zone de chevauchement (15) annulaire dans l'état implanté.

4. Dispositif d'occlusion selon l'une des revendications précédentes,
**caractérisé en ce que**
les tronçons de paroi (12, 13) comprennent un treillis de fils (19).

5. Dispositif d'occlusion selon la revendication 4,
**caractérisé en ce que**
les tronçons de paroi (12, 13) présentent respectivement des premiers fils (19a) qui sont dirigés de façon centrale dans un centre (21) du tronçon de paroi (12, 13), sont écartés hors du plan de la paroi et reliés à l'élément de liaison (14).

6. Dispositif d'occlusion selon la revendication 5,
**caractérisé en ce que**
les premiers fils (19a) sont dirigés hors de ou à l'intérieur de la cavité (11).

7. Dispositif d'occlusion selon l'une des revendications 4 à 6,
**caractérisé en ce que**
les fils (19), en particulier les premiers fils (19a), d'au moins un tronçon de paroi (12, 13) forment l'élément de liaison (14).

8. Dispositif d'occlusion selon l'une des revendications 4 à 7,
**caractérisé en ce que**
les tronçons de paroi (12, 13) présentent respectivement des seconds fils (19b) qui sont déviés au centre (21) du tronçon de paroi (12, 13) dans le plan de la paroi et qui forment respectivement une boucle (20).

9. Dispositif d'occlusion selon l'une des revendications précédentes,
**caractérisé en ce que**
l'élément de liaison (14) est disposé en intégralité à l'intérieur de la paroi (10).

10. Dispositif d'occlusion selon l'une des revendications précédentes,
**caractérisé en ce que**
l'élément de liaison (14) est conçu à la manière d'un ressort.

11. Dispositif d'occlusion selon l'une des revendications précédentes,
**caractérisé en ce que**
l'élément de liaison (14) présente un élément extensible (17) qui est disposé en intégralité dans la cavité (11).

12. Dispositif d'occlusion selon la revendication 11,
**caractérisé en ce que**
l'élément extensible (17) présente un treillis.

13. Dispositif d'occlusion selon l'une des revendications précédentes,
**caractérisé en ce**
**qu'**un des tronçons de paroi (12, 13) est relié au centre à un élément de recouvrement (18) qui est disposé dans la cavité (11) et repose de façon détachée sur le tronçon de paroi (12, 13) à l'état de repos.

14. Agencement comprenant un dispositif d'occlusion selon l'une revendications des précédentes et un fil de transport qui est relié de façon séparable au dispositif d'occlusion et est disposé mobile longitudinalement à l'intérieur d'une enveloppe de cathéter de telle sorte que le dispositif d'occlusion comprimé peut être dirigé par l'enveloppe de cathéter sur un lieu de traitement.
